# EUROPEAN PATENT SPECIFICATION

(11) **EP 0 815 856 B1**
(45) Date of publication and mention of the grant of the patent: **01.06.2005**
(21) Application number: 97105351.7
(22) Date of filing: 18.11.1991
(51) Int. Cl.: A61K 31/17, A61K 31/47, A61K 31/44, A61K 31/535, A61K 31/505, A61K 31/34, A61K 31/415, A61K 31/495, A61K 31/215, A61K 31/18, A61K 31/325, A61K 31/40, A61K 31/195

(54) **Retroviral protease inhibitors**
Retrovirusprotease Inhibitoren
Inhibiteurs de protéases rétrovirales

(30) Priority: 19.11.1990 US 615210
(43) Date of publication of application: 07.01.1998
(62) Divisional of application: 92901068.4
(73) Proprietor: MONSANTO COMPANY, St. Louis, Missouri 63167 (US); G.D. Searle & Co., Skokie, Illinois 60077 (US)
(72) Inventor: Clare, Michael, Skokie, Illinois 60077 (US); Freskos, John Nicholas, Clayton, Missouri 63105 (US); Heintz, Robert Martin, Ballwin, Missouri 63021 (US); Decrescenzo, Gary Anthony, St. Peters, Missouri 63376 (US); Getman, Daniel Paul, Chesterfield, MO. 63107 (US); Lin, Ko-Chung, St. Louis, Missouri 63146 (US); Mueller, Richard August, Glencoe, Illinois 60022 (US); Talley, John Jeffrey, Brebtwood, Mo.63144-1818 (US); Sun, Eric Tak On, Buffalo Grove, IL 60039 (US); Reed, Katryn Lea, Richmond Heights, Missouri 63117 (US); Vasquez, Michael Lawrence, Gurnee, Illinois 60031 (US)
(74) Representative: Colens, Alain

(56) References cited:
- EP-A- 0 356 223
- FR-A- 2 620 451

## Description

### BACKGROUND OF THE INVENTION

### 1. Field of the Invention

The present invention relates to retroviral protease inhibitors and, more particularly, relates to novel compounds and a composition and method for inhibiting retroviral proteases. This invention, in particular, relates to urea-containing hydroxyethylamine protease inhibitor compounds, a composition and method for inhibiting retroviral proteases such as human immunodeficiency virus (HIV) protease and for treating a retroviral infection, e.g., an HIV infection. The subject invention also relates to processes for making such compounds as well as to intermediates useful in such processes.

### 2. Related Art

During the replication cycle of retroviruses, gag and gag-pol gene products are translated as proteins. These proteins are subsequently processed by a virally encoded protease (or proteinase) to yield viral enzymes and structural proteins of the virus core. Most commonly, the gag precursor proteins are processed into the core proteins and the pol precursor proteins are processed into the viral enzymes, e.g., reverse transcriptase and retroviral protease. It has been shown that correct processing of the precursor proteins by the retroviral protease is necessary for assembly of infectious virons. For example, it has been shown that frameshift mutations in the protease region of the pol gene of HIV prevents processing of the gag precursor protein. It has also been shown through site-directed mutagenesis of an aspartic acid residue in the HIV protease that processing of the gag precursor protein is prevented. Thus, attempts have been made to inhibit viral replication by inhibiting the action of retroviral proteases.

Retroviral protease inhibition typically involves a transition-state mimetic whereby the retroviral protease is exposed to a mimetic compound which binds (typically in a reversible manner) to the enzyme in competition with the gag and gag-pol proteins to thereby inhibit replication of structural proteins and, more importantly, the retroviral protease itself. In this manner, retroviral replication proteases can be effectively inhibited.

Several classes of mimetic compounds have been proposed, particularly for inhibition of proteases, such as for inhibition of HIV protease. Such mimetics include hydroxyethylamine isosteres and reduced amide isosteres. See, for example, EP O 346 847; EP O 342,541; Roberts et al, "Rational Design of Peptide-Based Proteinase Inhibitors, "Science, 248, 358 (1990); and Erickson et al, "Design Activity, and 2.8Å Crystal Structure of a C₂ Symmetric Inhibitor Complexed to HIV-1 Protease," Science, 249, 527 (1990).

Several classes of mimetic compounds are known to be useful as inhibitors of the proteolytic enzyme renin. See, for example, U.S. No. 4,599,198; U.K. 2,184,730; G.B. 2,209,752; EP O 264 795; G.B. 2,200,115 and U.S. SIR H725. Of these, G.B. 2,200,115, GB 2,209,752, EP O 264,795, U.S. SIR H725 and U.S. 4,599,198 disclose urea-containing hydroxyethylamine renin inhibitors. However, it is known that, although renin and HIV proteases are both classified as aspartyl proteases, compounds which are effective renin inhibitors generally cannot be predicted to be effective HIV protease inhibitors.

### BRIEF DESCRIPTION OF THE INVENTION

The present invention is directed to virus inhibiting compounds and compositions. More particularly, the present invention is directed to retroviral protease inhibiting compounds and compositions, to a method of inhibiting retroviral proteases, to processes for preparing the compounds and to intermediates useful in such processes. The subject compounds are characterized as urea-containing hydroxyethylamine inhibitor compounds.

### DETAILED DESCRIPTION OF THE INVENTION

In accordance with the present invention, there is provided the use of a compound represented by the formula: 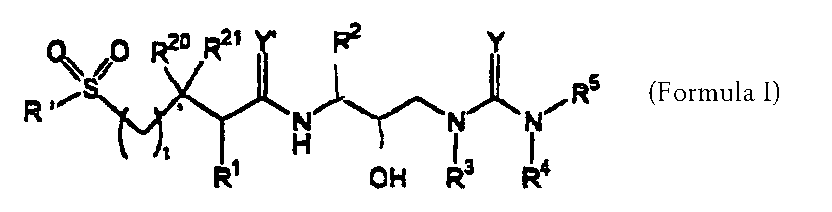 for preparing a medicament for inhibiting a retroviral protease, for treating a retroviral infection and for treating AIDS wherein:
R' represents radicals as defined for R³;
R¹ represents hydrogen, -CH₂SO₂NH₂, alkyl and cycloalkyl radicals and amino acid side chains selected from asparagine, S-methyl cysteine and the corresponding sulfoxide and sulfone derivatives thereof, glycine, alloisoleucine, alanine, leucine, tert-leucine, phenylalanine, ornithine, threonine, allo-threonine, isoleucine, histidine, norleucine, valine, serine, aspartic acid, beta-cyano alanine side chains;
R² represents alkyl, aryl, cycloalkyl, cycloalkylalkyl and aralkyl radicals optionally substituted with a group selected from -OR⁹, -SR⁹, and halogen radicals, wherein R⁹ represents hydrogen and alkyl radicals;
R³ represents alkyl, alkenyl, hydroxyalkyl, cycloalkyl, cycloalkylalkyl, heterocycloalkyl, heterocycloalkylalkyl, aryl, heteroaryl, aralkyl, and heteroaralkyl radicals;
R⁴ and R⁵, independently represent hydrogen and radicals as defined by R³, or R⁴ and R⁵ together with the nitrogen atom to which they are bonded represent heterocycloalkyl and heteroatyl radicals;
R²⁰ and R²¹ represnt radicals as defined for R¹; and
Y and Y' independently represent O and S
t represent 0 or 1; and
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom,
- heteroaryl is an aromatic monocyclic, bicyclic, or tricyclic heterocycle which contains the hetero atoms and is optionally substituted as defined above with respect to the definition of heterocycloalkyl,
- heteroaralkyl is a alkyl radical having 1 to 10 carbon atoms in which one hydrogen is replaced by a heteroaryl.

Preferably, Y and Y' represent O.

Preferably, R³ represents radicals as defined above which contain no α-branching, e.g., as in an isopropyl radical or a t-butyl radical. The preferred radicals are those which contain a -CH₂- moiety between the nitrogen of the urea and the remaining portion of the radical. Such preferred groups include, but are not limited to, benzyl, isobutyl, n-butyl, isoamyl and cyclohexylmethyl.

As utilized herein, the term "alkyl", alone or in combination, means a straight-chain or branched-chain alkyl radical containing from 1 to 10, preferably from 1 to 8, carbon atoms. Examples of such radicals include methyl, ethyl, n-propyl, isopropyl, n-butyl, isobutyl, sec-butyl, tert-butyl, pentyl, isoamyl, hexyl and octyl . The term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy and tert-butoxy . The term "cycloalkyl" means an alkyl radical which contains from about 3 to 8 carbon atoms and is cyclic. The term "cycloalkylalkyl" means an alkyl radical as defined above which is substituted by a cycloalkyl radical containing from about 3 to 8, preferably from about 3 to 6, carbon atoms. Examples of such cycloalkyl radicals include cyclopropyl, cyclobutyl, cyclopentyl and cyclohexyl . The term "aryl", alone or in combination, means a phenyl or naphthyl radical which optionally carries one or more substituents selected from alkyl, alkoxy, halogen, hydroxy, amino and nitro, such as phenyl, p-tolyl, 4-methoxyphenyl, 4-(tert-butoxy)phenyl, 4-fluorophenyl, 4-chlorophenyl, 4-hydroxyphenyl, 1-naphthyl and 2-naphthyl. The term "aralkyl", alone or in combination, means an alkyl radical as defined above in which one hydrogen atom is replaced by an aryl radical as defined above, such as benzyl and 2-phenylethyl. The term "aralkoxy carbonyl", alone or in combination, means a radical of the formula -C(O)-O-aralkyl in which the term "aralkyl" has the significance given above. An example of an aralkoxycarbonyl radical is benzyloxycarbonyl. The term "leaving group" generally refers to groups readily displaceable by a nucleophile, such as an amine, a thiol or an alcohol nucleophile. Such leaving groups are well known and include carboxylates, N-hydroxysuccinimide, N-hydroxybenzotriazole, halides, triflates, tosylates -OR and -SR and the like. Preferred leaving groups are indicated herein where appropriate.

Procedures for preparing the compounds of Formula I are set forth below. It should be noted that the general procedure is shown as it relates to preparation of compounds having the specified stereochemistry, namely wherein the stereochemistry about the hydroxyl group is designated as (R).

### Preparation of Compounds of Formula I

The compounds of the present invention represented by Formula II above can be prepared utilizing the following general procedure. An N-protected chloroketone derivative of an amino acid having the formula: 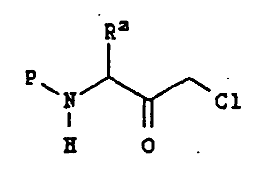 wherein P represents an amino protecting group, and R² is as defined above, is reduced to the corresponding alcohol utilizing an appropriate reducing agent. Suitable amino protecting groups are well known in the art and include carbobenzoxy, butyryl, t-butoxycarbonyl, acetyl, benzoyl and the like. A preferred amino protecting group is carbobenzoxy. A preferred N-protected chloroketone is N-benzyloxycarbonyl-L-phenylalanine chloromethyl ketone. A preferred reducing agent is sodium borohydride. The reduction reaction is conducted at a temperature of from -10°C to about 25°C, preferably at about 0°C, in a suitable solvent system such as, for example, tetrahydrofuran, and the like. The N-protected chloroketones are commercially available from Bachem, Inc., Torrance, California. Alternatively, the chloroketones can be prepared by the procedure set forth in S. J. Fittkau, J. Prakt. Chem., 315, 1037 (1973), and subsequently N-protected utilizing procedures which are well known in the art.

The resulting alcohol is then reacted, preferably at room temperature, with a suitable base in a suitable solvent system to produce an N-protected amino epoxide of the formula: 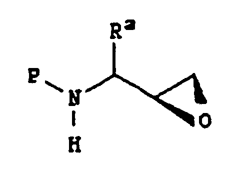 wherein P and R² are as defined above. Suitable solvent systems for preparing the amino epoxide include ethanol, methanol, isopropanol, tetrahydrofuran, dioxane, and the like including mixtures thereof. Suitable bases for producing the epoxide from the reduced chloroketone include potassium hydroxide, sodium hydroxide, potassium t-butoxide, DBU and the like. A preferred base is potassium hydroxide.

The amino epoxide is then reacted, in a suitable solvent system, with an equal amount, or preferably an excess of, a desired amine of the formula:

R³NH₂

wherein R³ is hydrogen or is as defined above. The reaction can be conducted over a wide range of temperatures, e.g., from about 14°C to about 100°C, but is preferably, but not necessarily, conducted at a temperature at which the solvent begins to reflux. Suitable solvent systems include those wherein the solvent is an alcohol, such as methanol, ethanol, isopropanol, and the like, ethers such as tetrahydrofuran, dioxane and the like, and toluene, N,N-dimethylformamide, dimethyl sulfoxide, and mixtures thereof. A preferred solvent is isopropanol. Exemplary amines corresponding to the formula R³NH₂ include benzyl amine, isobutylamine, n-butyl amine, isopentyl amine, isoamylamine, cyclohexanemethyl amine, naphthylene methyl amine and the like. The resulting product is a 3-(N-protected amino)-3-(R²)-1-(NHR³)-propan-2-ol derivative (hereinafter referred to as an amino alcohol) can be represented by the formula: 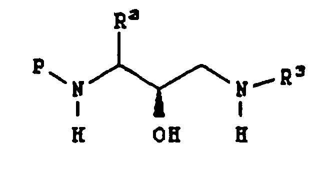 wherein P, R² and R³ are as described above.

For producing compounds of Formula I wherein R⁵ is hydrogen, the resulting amino alcohol described above is then reacted, in a suitable solvent system, with an isocyanate of the formula R⁴NCO wherein R⁴ is as defined above. Where Y in Formula I above is sulfur, the resulting amino alcohol is reacted with an isothiocyanate of the formula R⁴NCS under similar conditions. Suitable solvent systems include tetrahydrofuran, methylene chloride, and the like and mixtures thereof. The resulting product is a urea derivative of the amino alcohol or the corresponding sulfur analog thereof and can be represented by the formula: 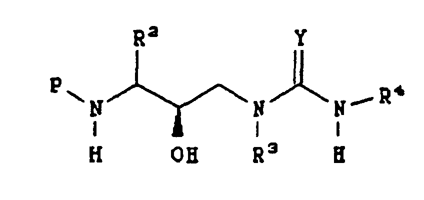 wherein P, Y, R², R³ and R⁴ are as defined above. The isocyanates of the formula R⁴NCO can be prepared by the reaction of an amine (R³NH₂) with phosgene, triphosgene, carbodiimidazole, or carbonate ((RO)₂CO) under conditions well-known in the art. The isothiocyanates of the formula R₄NCS can be prepared by similar procedures, e.g., reaction of the amine with thiophosgene, which are also well known in the art. In addition, the isocyanates and isothiocyanates are commercially available from Aldrich Chemical Company.

For preparing compounds of Formula I wherein R⁵ is other than hydrogen, the resulting amino alcohol described above is then reacted, in a suitable solvent system, with a compound represented by the formula: 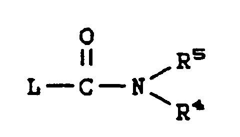 wherein R⁴ and R⁵ are as described above and L represents a leaving group such as a halide, e.g., chloride, imidazole radical, the radical p-NO₂-(C₆H₄)-O-, and the like. A preferred compound represented by this formula is a carbamoyl chloride. The corresponding sulfur analogs can be utilized where Y of Formula I is S.

The urea derivative of the amino alcohol and the corresponding sulfur analog can be represented by the formula:

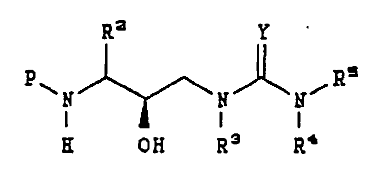

Following preparation of the urea derivative, or corresponding analogs wherein Y is S, the amino protecting group P is removed under conditions which will not affect the remaining portion of the molecule. These methods are well known in the art and include acid hydrolysis, hydrogenolysis and the like. A preferred method involves removal of the protecting group, e.g., removal of a carbobenzoxy group, by hydrogenolysis utilizing palladium on carbon in a suitable solvent system such as an alcohol, acetic acid, and the like or mixtures thereof. Where the protecting group is a t-butoxycarbonyl group, it can be removed utilizing an inorganic or organic acid, e.g., HCl or trifluoroacetic acid, in a suitable solvent system, e.g., dioxane or methylene chloride. The resulting product is the amine salt derivative. Following neutralization of the salt, the amine is then further reacted with a free acid prepared as follows.

A mercaptan of the formula R'SH is reacted with a substituted methacrylate of the formula: 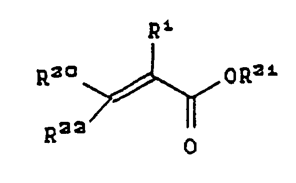 by way of a Michael Addition. The Michael Addition is conducted in a suitable solvent and in the presence of a suitable base, to produce the corresponding thiol derivative represented by the formula: 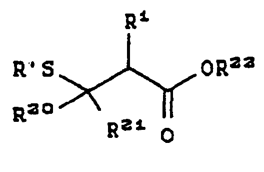 wherein R' and R¹ represent radicals defined above; R²⁰ and R²¹ represent hydrogen and radicals as defined for R¹; and R²² represents radicals as defined by R³. Suitable solvents in which the Michael Addition can be conducted include alcohols such as, for example, methanol, ethanol, butanol and the like, as well as ethers, e.g., THF, and acetonitrile, DMF, DMSO, and the like, including mixtures thereof. Suitable bases include Group I metal alkoxides such as, for example sodium methoxide, sodium ethoxide, sodium butoxide and the like as well as Group I metal hydrides, such as sodium hydride, including mixtures thereof.

The thiol derivative is converted into the corresponding sulfone of the formula: 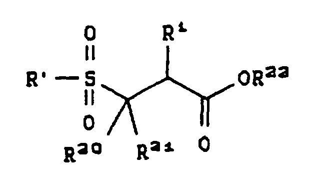 by oxidizing the thiol derivative with a suitable oxidation agent in a suitable solvent. Suitable oxidation agents include, for example, hydrogen peroxide, sodium meta-perborate, oxone (potassium peroxy monosulfate), meta-chloroperoxybenzoic acid, and the like, including mixtures thereof. Suitable solvents include acetic acid (for sodium meta-perborate) and, for other peracids, ethers such as THF and dioxane, and acetonitrile, DMF and the like, including mixtures thereof.

The sulfone is then converted to the corresponding free acid of the formula: 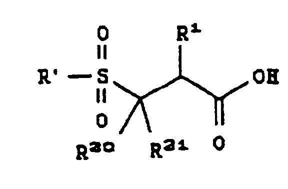 utilizing a suitable base, e.g., lithium hydroxide, sodium hydroxide, and the like, including mixtures thereof, in a suitable solvent, such as, for example, THF, acetonitrile, DMF, DMSO, methylene chloride and the like, including mixtures thereof.

The free acid is then coupled, utilizing, as described above, procedures well known in the art, to the urea derivative, or analog thereof, of an amino alcohol which is described above . The resulting product is a compound represented by Formula I.

Alternatively, one can couple the urea isostere to the commercially available acid, 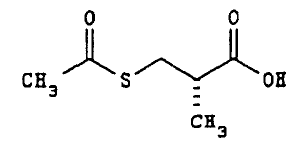 remove the thioacetyl group with a suitable base, such as hydroxide, or an amine, such as ammonia, and then react the resulting thiol with an alkylating agent, such as an alkyl halide, tosylate or mesylate to afford compounds at the following structure:

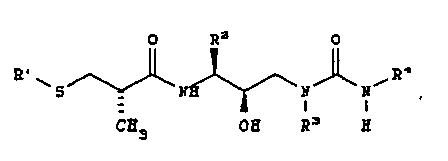

The sulfur can then be oxidized to the corresponding sulfone using suitable oxidizing agents, as described above, to afford the desired compounds of the following structure: 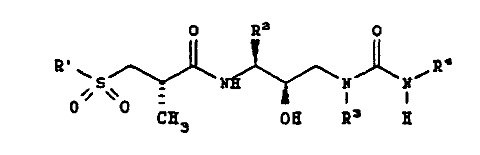 Alternatively, to prepare compounds of Formula I, a substituted methacrylate of the formula:

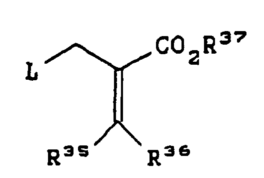

wherein L represents a leaving group as previously defined, R³⁵ and R³⁶ represent hydrogen and radicals as defined for R¹; and R³⁷ represents alkyl, aralkyl, cycloalkyl and cycloalkylalkyl radicals,
is reacted with a suitable sulfonating agent, such as, for example, a sulfinic acid represented by the formula R'SO₂M, wherein R' represents radicals as defined above and M represents a metal adapted to form a salt of the acid, e.g., sodium, to produce the corresponding sulfone represented by the formula: 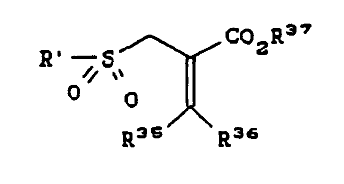 wherein R', R³⁵, R³⁶ and R³⁷ are as defined above. The sulfone is then hydrolyzed in the presence of a suitable base, such as lithium hydroxide, sodium hydroxide and the like, to the compound represented by the formula: 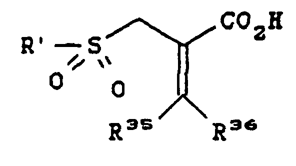 wherein R', R³⁵ and R³⁶ represent radicals as defined above. The resulting compound is then asymmetrically hydrogenated utilizing an asymmetric hydrogenation catalyst such as, for example, a ruthenium-BINAP complex, to produce the reduced product, substantially enriched in the more active isomer, represented by the formula: 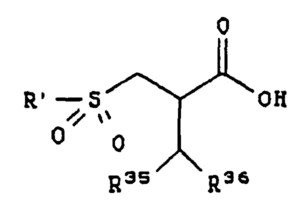 wherein R', R³⁵ and R³⁶ represent radicals as defined above. Where the more active isomer has the R-stereochemistry, a Ru(R-BINAP) asymmetric hydrogenation catalyst can be utilized. Conversely, where the more active isomer has the S-sterochemistry, a Ru(S-BINAP) catalyst can be utilized. Where both isomers are active, or where it is desired to have a mixture of the two diastereomers, a hydrogenation catalyst such as platinum, or palladium, on carbon can be utilized to reduce the above compound. The reduced compound is then coupled to the urea isostere, as described above, to produce compounds of Formula I.

It is contemplated that where R³ of the amino alcohol intermediate is hydrogen, the inhibitor compounds can be prepared through reductive amination of the final product of the reaction between the amino alcohol and the amine or at any other stage of the synthesis for preparing the inhibitor compounds.

Contemplated equivalents of the general formulas set forth above for the antiviral compounds and derivatives as well as the intermediates are compounds otherwise corresponding thereto and having the same general properties wherein one or more of the various R groups are simple variations of the substituents as defined therein, e.g., wherein R is a higher alkyl group than that indicated. In addition, where a substituent is designated as, or can be, a hydrogen, the exact chemical nature of a substituent which is other than hydrogen at that position, e.g., a hydrocarbyl radical or a halogen, hydroxy, amino and the like functional group, is not critical so long as it does not adversely affect the overall activity and/or synthesis procedure.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

All reagents were used as received without purification. All proton and carbon NMR spectra were obtained on either a Varian VXR-300 or VXR-400 nuclear magnetic resonance spectrometer.

### Example 1A

The procedure described below illustrates preparation of compounds of Formula I. Propanamide, N-[3-[[[(1,1-dimethylethyl)amino]carbonyl](3-methylbutyl)amino]-2-hydroxy-1-(phenylmethyl)propyl]-2-methyl-3-(2-phenylethylsulfonyl)-,[1S-[1R*(R*),2S*]] and its diastereomer.

### Part A

A solution of methyl methacrylate (7.25 g, 72.5 mmol) and phenethyl mercaptan (10.0 g, 72.5 mmol) in 100 mL of methanol was cooled in an ice bath and treated with sodium methoxide (100 mg, 1.85 mmol). The solution was stirred under nitrogen for 3 h and then concentrated in vacuo to give an oil that was taken up in ether and washed with 1 N aqueous potassium hydrogen sulfate, saturated aqueous sodium chloride, dried over anhydrous magnesium sulfate, filtered and concentrated to give 16.83 g, 97.5% of methyl 2-(R,S)-methyl-4-thia-6-phenyl hexanoate as an oil. TLC on SiO₂ eluting with 20:1 hexane:ethyl acetate (v:v) R_{f}=0.41.

### Part B

A solution of methyl 2-(R,S)-methyl-4-thia-6-phenyl hexanoate (4.00 g, 16.8 mmol) in 100 mL of dichloromethane was stirred at room temperature and treated portion wise with meta-chloroperoxybenzoic acid (7.38 g, 39.2 mmol) over approximately 40 m. The solution was stirred at room temperature for 16 h and then filtered and the filterate washed with saturated aqueous sodium bicarbonate, 1N sodium hydroxide, saturated aqueous sodium chloride, dried over anhydrous magnesium sulfate, filtered, and concentrated to give 4.50 g, 99% of desired sulfone. The unpurified sulfone was dissolved in 100 mL of tetrahydrofuran and treated with a solution of lithium hydroxide (1.04 g, 24.5 mmol) in 40 mL of water. The solution was stirred at room temperature for 2 m and then concentrated *in vacuo*. The residue was then acidified with 1N aqueous potassium hydrogen sulfate to pH=1 and then extracted three times with ethyl acetate. The combined ethyl acetate solution was washed with saturated aqueous sodium chloride, dried over anhydrous magnesium sulfate, filtered and concentrated to give a white solid. The solid was taken up in boiling ethyl acetate/hexane and allowed to stand undisturbed whereupon white needles formed that were isolated by filtration and air dried to give 3.38 g, 79% of 2-(R,S)-methyl-3(β-phenethylsulfonyl)-propionic acid, mp 91-93°C.

### Part C

A solution of 2-(R,S)-methyl-3(β-phenethylsulfonyl)-propionic acid (166.1 mg, 0.65 mmol), N-hydroxybenzotriazole (HOBT) (146.9 mg, 0.97 mmol), and 1-(3-dimethylaminopropyl)-3-ethylcarbodiimide hydrochloride (EDC) (145.8 mg, 0.75 mmol) in 4 mL of anhydrous dimethylformamide (DMF) cooled to 0°C and stirred under nitrogen for 0.5 h. This solution was then treated with 3-[[(dimethylethyl)amino]carbonyl](3-methylbutyl)amino-2(R)-hydroxy-1(S)-(phenylmethyl)propyl amine (201.9 mg, 0.59 mmol) and stirred at room temperature for 16 h. The solution was poured into 30 mL of 60% saturated aqueous sodium bicarbonate solution. The aqueous solution was then decanted from the organic residue. The organic residue was taken up in dichloromethane and washed with 10% aqueous citric acid, brine, dried over anhydrous magnesium sulfate, filtered and concentrated to give 110.0 mg, 32% of (2R,3S)-3-[N-2-(R)-methyl-3-(β-phenethylsulfonyl)propionyl]amido-1-isoamyl-1-(tert-butylcarbamoyl)amino-4-phenyl-2-butanol and (2R,3S)-3-[N-2-(S)-methyl-3-(β-phenethylsulfonyl)propionyl]amido-1-isoamyl-1-(tert-butylcarbamoyl)amino-4-phenyl-2-butanol, FAB mass spectrum (MH+) =588. Flash chromatography of the mixture on silica gel eluting with 1:1 hexane:ethyl acetate afforded the separated diastereomers.

### Example 1B

Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)- [1S-[1R*(R*), 2S*]], and its diastereomer.

### Part A

A solution of methyl 2-(bromomethyl)-acrylate (26.4 g, 0.148 mol) in 100 mL of methanol was treated with sodium methanesulfinate (15.1 g, 0.148 mol) portion wise over 10 m at room temperature. The solution was then stirred at room temperature for a period of 1.25 h and the solution concentrated in vacuo. The residue was then taken up in water and extracted four times with ethyl acetate. The combined ethyl acetate solution was washed with saturated sodium chloride, dried over anhydrous magnesium sulfate, filtered and concentrated to give a white solid, 20.7 g which was taken up in boiling acetone/methyl tert-butyl ether and allowed to stand whereupon crystals of pure methyl 2-(methylsulfonylmethyl) acrylate 18.0 g, 68% formed, mp 65-68 0°C.

### Part B

A solution of methyl 2-(methylsulfonylmethyl) acrylate (970 mg, 5.44 mmol) in 15 mL of tetrahydrofuran was treated with a solution of lithium hydroxide (270 mg, 6.4 mmol) in 7 mL of water. The solution was stirred at room temperature for 5 m and then acidified to pH=1 with 1 N aqueous potassium hydrogen sulfate and the solution extracted three times with ethyl acetate. The combined ethyl acetate solution was dried over anhydrous magnesium sulfate, filtered, and concentrated to give 793 mg, 89% of 2-(methylsulfonylmethyl) acrylic acid, mp 147-149 0°C.

### Part C

A solution of 2-(methylsulfonylmethyl) acrylic acid (700 mg, 4.26 mmol) in 20 mL of methanol was charged into a Fisher-Porter bottle along with 10% palladium on carbon catalyst under a nitrogen atmosphere. The reaction vessel was sealed and flushed five times with nitrogen and then five times with hydrogen. The pressure was maintained at 50 psig for 16 h and then the hydrogen was replaced with nitrogen and the solution filtered through a pad of celite to remove the catalyst and the filterate concentrated *in vacuo* to give 682 mg 96% of 2-(R,S)-methyl-3-methylsulfonyl propionic acid.

### Part D

A solution of 2-(R,S)-methyl-3(methylsulfonyl) propionic acid (263.5 mg, 1.585 mmol), N-hydroxybenzotriazole (HOBT) (322.2 mg, 2.13 mmol), and 1-(3-dimethylaminopropyl)-3-ethylcarbodiimide hydrochloride (EDC) (339.1 mg, 1.74 mmol) in 4 mL of anhydrous dimethylformamide (DMF) cooled to 0°C and stirred under nitrogen for 0.5 h. This solution was then treated with 3-[[(1,1-dimethylethyl)amino]carbonyl](3-methylbutyl)amino-2(R)-hydroxy-I(S)-(phenylmethyl)propyl amine (543.5 mg, 1.58 mmol) and stirred at room temperature for 16 h. The solution was poured into 60 mL of 60% saturated aqueous sodium bicarbonate solution. The aqueous solution was then decanted from the organic residue. The organic residue was taken up in dichloromethane and washed with 10% aqueous citric acid, brine, dried over anhydrous magnesium sulfate, filtered and concentrated to give 471.8 mg, 60% of Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)-, [1S-[1R*(R*), 2S*]]- and its diastereomer.

### Example 2

### Preparation of Sulfone Inhibitors From L-(+)-S-acetyl-β-mercaptoisobutyric Acid

### Part A:

Propanamide, N-[3-[[[(1,1-dimethylethyl)amino]carbonyl](3-methylbutyl)amino]-2-hydroxy-1-(phenylmethyl)propyl]-2-methyl-3-S-acetyl)-[1S-[1R*),2S*]]-.

A round-bottomed flask was charged with (2R,3R)-3-amino-1-isoamyl-1-(tert-butylcarbamoyl)amino-4-phenyl-2-butanol (901.5 mg, 2.575 mmol), L-(+)-S-acetyl-b-mercaptoisobutyric acid (164.5 mg, 2.575 mmol), 1-(3-dimethylaminopropyl)-3-ethylcarbodiimide hydrochloride (EDC) (339.1 mg, 1.74 mmol), and 10 mL of CH₂Cl₂ and allowed to stir at room temperature for 16 h. The solution was concentrated in vacuo and the residue taken up in ethyl acetate, washed with 1N KHSO₄ sat. aq. NaHCO₃, brine, dried over anhydrous MgSO₄, filtered and concentrated to give an oil that was purified by radial chromatography on SiO₂ eluting with ethyl acetate to give the pure product, 800 mg, 63%.

### Part B:

Propanamide, N-[3-[[[1,1-dimethylethyl)amino]carbonyl](3-methylbutyl)amino]-2-hydroxy-1-(phenylmethyl)propyl]-2-methyl-3-mercapto)-, [1S-[1R*(R*),2S*]]-.

A solution of [1S-[1R*(R*),2S*]]- N-[3-[[[(1,1-dimethylethyl)amino]carbonyl](3-methylbutyl)amino]-2-hydroxy-1-(phenylmethyl)propyl]-2-methyl-3-S-acetyl)-propanamide (420 mg, 0.85 mmol) in 10 mL of methanol was treated with anhydrous ammonia for ca. 1 m at 0°C. The solution was stirred at that temperature for 16 h and then concentrated in vacuo to give 380 mg, 99%, of the desired product that was used directly in the next step without further purification.

### Part C:

Propanamide, N-[3-[[[(1,1-dimethylethyl)amino]carbonyl](3-methylbutyl)amino]-2-hydroxy-1-(phenylmethyl)propyl]-2-methyl-3-S-methyl-, [1S-[1R*(R*),2S*]]-.

A solution of [1S-[1R*(R*),2S*]]- N-[3-[[[(1,1-dimethylethyl)amino]carbonyl](3-methylbutyl)amino]-2-hydroxy-1-(phenylmethyl)propyl]-2-methyl-3-mercapto)-propanamide (380 mg, 0.841 mmol) in 10 mL of dry toluene under nitrogen was treated in rapid succession with 1,8-diazabicyclo[5.4.0]undec-7-ene, (DBU), (128.1 mg. 0.841 mmol) and iodomethane (119.0 mg, 0.841 mmol). After 0.5 h at room temperature the reaction was found to be complete and the solution was diluted with ethyl acetate washed with 1N KHSO₄, sat. aq. NaHCO₃, brine. After the solution was dried over anhydrous MgSO₄, filtered and concentrated in vacuo the desired product was obtained as white foam was obtained, 370 mg, 94.5%, that was used directed in the next step.

### Part D:

Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)-, [1S-[1R*(R*),2S*]]-.

A solution of [1S-[1R*(R*),2S*]]-N-[3-[[[(1,1-dimethylethyl)amino]carbonyl](3-methylbutyl)amino]-2-hydroxy-1-(phenylmethyl)propyl]-2-methyl-3-S-methyl)-propanamide (340 mg, 0.73 mmol) and sodium perborate (500 mg, 3.25 mmol) in 30 mL of glacial acetic acid was warmed to 55°C for 16 h. The solution was conentrated in vacuo and then the residue taken up in ethyl acetate, washed with water, sat. aq. NaHCO₃, brine, dried over anhydrous MgSO₄, filtered and concentrated to give the desired product as a white solid, 350 mg, 96%.

### Example 3

The compounds shown in Tables 1 and 2 were prepared generally according to the procedure set forth in Examples 1 and 2.

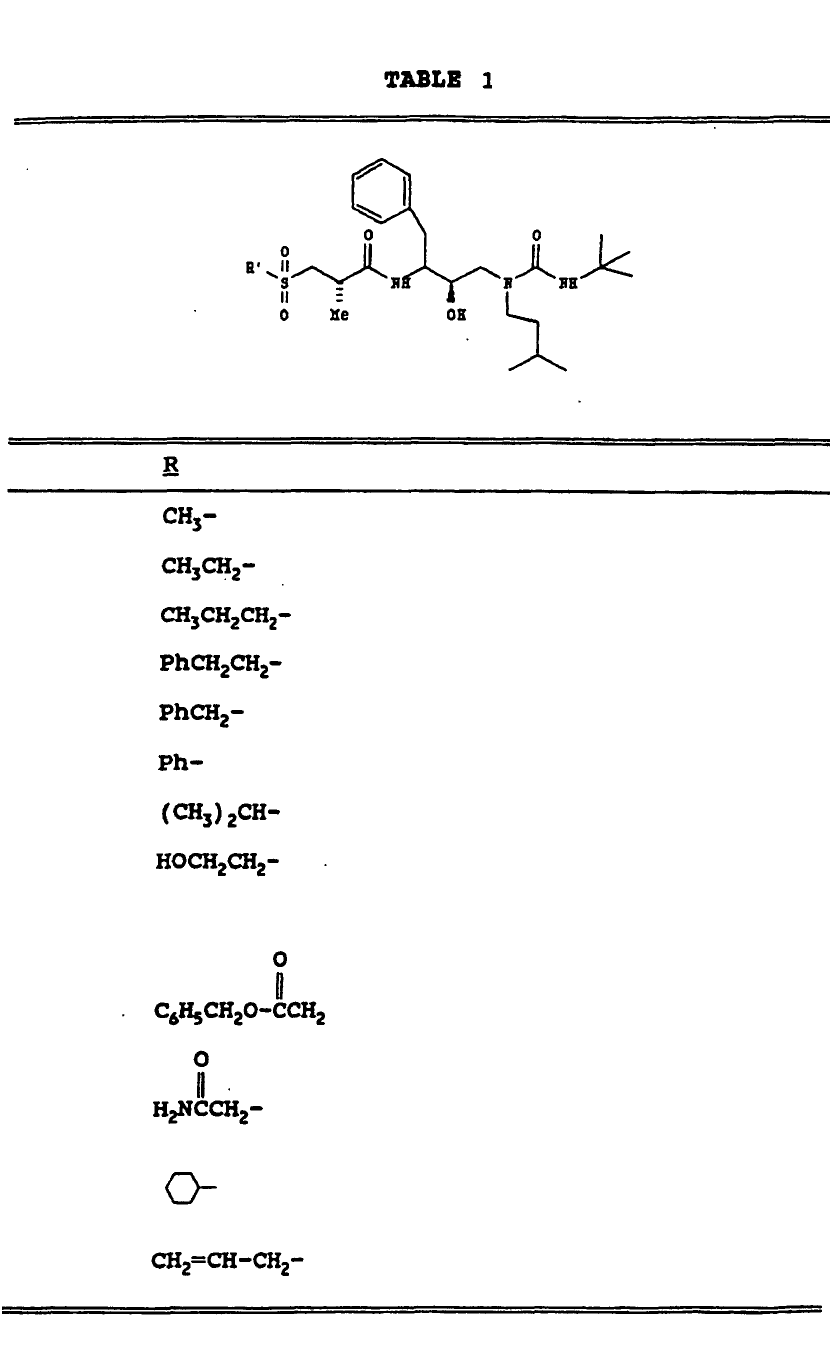

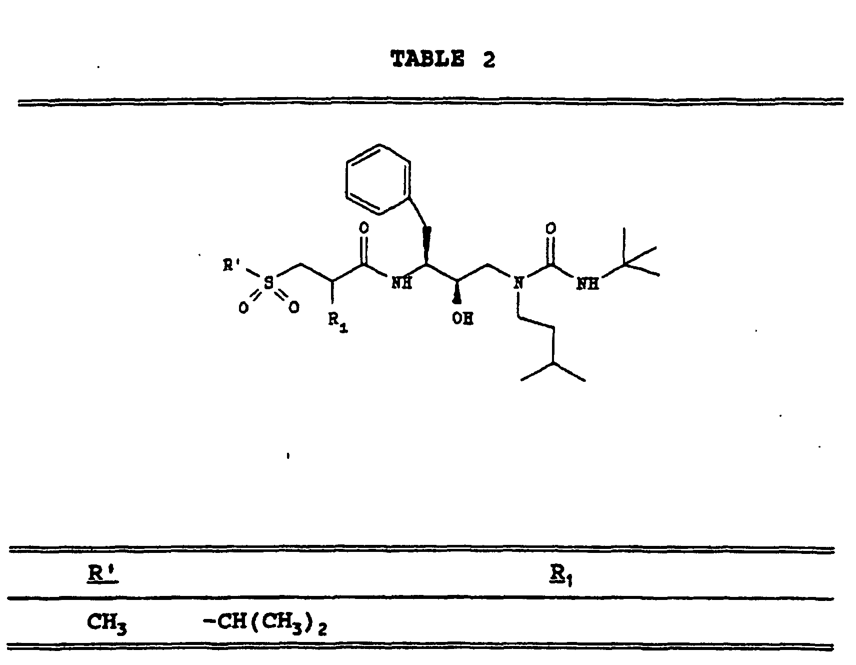

### Example 4

### Preparation of 2(S)-methyl-3-(methylsulfonyl)propionic Acid.

To a solution of 10g of D-(-)-S-benzoyl-b-mercaptioisobutyric acid t-butyl ester in 20 mL of methanol was bubbled in gaseous ammonia at 0°C. The reaction was allowed to then warm to room temperature, stirred overnight and concentrated under reduced pressure. The resulting mixture of a solid (benzamide) and liquid was filtered to provide 5.21g of a pale oil which then solidified. This was identified as 2(S)-methyl-3-mercaptopropionic aid t-butyl ester.

To a solution of 5.21g of 2(S)-methyl-3-mercaptopropionic acid t-butyl ester in 75 mL of toluene at 0°C was added 4.50g of 1,8-diazabicyclo[5.40]undec-7-ene and 1.94 mL of methyl iodide. After stirring at room temperature for 2.5 hours, the volatiles were removed, ethyl acetate added, washed with dilute hydrochloric acid, water, brine, dried and concentrated to afford 2.82g of a pale oil, identified as 2(S)-methyl-3-(thiomethyl)propionic acid t-butyl ester.

To a solution of 2.82g of 2(S)-methyl-3-(thiomethyl)propionic acid t-butyl ester in 50 mL of acetic acid was added 5.58g of sodium perborate and the mixture heated to 55°C for 17 hours. The reaction was poured into water, extracted with methylene chloride, washed with aqueous sodium bicarbonate, dried and concentrated to afford 2.68g of 2(S)-methyl-3-(methylsulfonyl)propionic acid t-butyl ester as a white solid.

To 2.68g of 2(S)-methyl-3-(methylsulfonyl)propionic acid t-butyl ester was added 20 mL of 4N hydrochloric acid/dioxane and the mixture stirred at room temperature for 19 hours. The solvent was removed under reduced pressure to afford 2.18g of crude product, which was recrystallized from ethyl acetate/hexane to yield 1.44g of 2(S)-methyl-3-(methylsulfonyl)propionic acid as white crystals.

### EXAMPLE 5

The compounds of the present invention are effective HIV protease inhibitors. Utilizing an enzyme assay as described below, the compounds set forth in the examples herein disclosed inhibited the HIV enzyme. The preferred compounds of the present invention and their calculated IC₅₀ (inhibiting concentration 50%, i.e., the concentration at which the inhibitor compound reduces enzyme activity by 50%) values are shown in Table 3 together with compounds of similar structure as claimed in divisional patent applications EP 97105350.9 (publication number EP813867) and 97 105352.5 (publication number EP813868). The enzyme method is described below. The substrate is 2-aminobenzoyl-Ile-Nle-Phe(p-NO₂)-Gln-ArgNH₂. The positive control is MVT-101 (Miller, M. et al, Science, 246, 1149 (1989)] The assay conditions are as follows:
- Assay buffer:: 20 mM sodium phosphate, pH 6.4
20% glycerol
1 mM EDTA
1 mM DTT
0.1% CHAPS

The above described substrate is dissolved in DMSO, then diluted 10 fold in assay buffer. Final substrate concentration in the assay is 80 µM.

HIV protease is diluted in the assay buffer to a final enzyme concentration of 12.3 nanomolar, based on a molecular weight of 10,780.

The final concentration of DMSO is 14% and the final concentration of glycerol is 18%. The test compound is dissolved in DMSO and diluted in DMSO to 10x the test concentration; 10µl of the enzyme preparation is added, the materials mixed and then the mixture is incubated at ambient temperature for 15 minutes. The enzyme reaction is initiated by the addition of 40µl of substrate. The increase in fluorescence is monitored at 4 time points (0, 8, 16 and 24 minutes) at ambient temperature. Each assay is carried out in duplicate wells.

**TABLE 3**

| Compound | | IC₅₀(nanomolar) |
|---|---|---|
| 1a) | Butanediamide, N¹-[3-[[[(1,1-dimethylethyl)amino]carbonyl] (2-methylpropyl)amino]-2-hydroxy-1-(phenylmethyl)propyl]-2-[(N-benzyloxycarbonyl)amino]-, [1S-[1R*(R*), 2S*]]- | 35 |
| 1b) | Butanediamide, N¹-[3-[[[(1,1-dimethylethyl)amino]carbonyl] (2-methylpropyl)amino]-2-hydroxy-1-(phenylmethyl)propyl]-2-[(2-quinolinylcarbonyl)amino]-, [1S-[1R*(R*),2S*]]- | 7.1 |
| 2a) | Butanediamide, N¹-[3-[[[(1,1-dimethylethyl)amino]carbonyl](3-methylbutyl)amino]-2-hydroxy-1-(phenylmethyl)propyl]-2-[(N-benzyloxycarbonyl)amino], [1S-[1R*(R*),2S*]]- | , 13.4 |
| 2b) | Butanediamide, N¹-[3-[[[(1,1-dimethylethyl)amino]carbonyl](3-methylbutyl)amino]-2-hydroxy-1-(phenylmethyl)propyl]-2-[(2-quinolinylcarbonyl)amino]-, [1S-[1R*(R*),2S*]]- | 3.2 |
| 3a) | Butanediamide, N¹-[3-[[[(1,1-dimethylethyl)amino]carbonyl](2-naphthylmethyl)amino]-2-hydroxy-1-(phenylmethyl)propyl]-2-[(N-benzyloxycarbonyl)amino]-, [1S-[1R*(R*), 2S*]]- | 31.0 |
| 3b) | Butanediamide, N¹-[3-[[[(1,1-dimethylethyl)amino]carbonyl](2-naphthylmethyl)amino]-2-hydroxy-1-(phenylmethyl)propyl]-2-[(2-quinolinylcarbonyl)amino]-, [1S-[1R*(R*),2S*]]- | 2.9 |
| 4a) | Butanediamide, N¹-[3-[[[(1,1-dimethylethyl)amino]carbonyl](2-phenylethyl)amino]-2-hydroxy-1-(phenylmethyl)propyl]-2-[(N-benzyloxycarbonyl)amino]-, [1S-[1R*(R*),2S*]]- | 70 |
| 4b) | Butanediamide, N¹-[3-[[[(1,1-dimethylethyl)amino]carbonyl] (2-phenylethyl)amino]-2-hydroxy-1-(phenylmethyl)propyl]-2-[(2-quinolinylcarbonyl)amino]-, [1S-[1R*(R*),2S*]]- | 10 |
| 5a) | Butanediamide, N¹-[3-[[[(1,1-dimethylethyl)amino]carbonyl](3,3-dimethylpropyl)amino]-2-hydroxy-1-(phenylmethyl)propyl]-2-[(N-benzyloxycarbonyl)amio], [1S-[1R*(R*),2S*]]- | 18 |
| 5b) | Butanediamide, N¹-[3-[[[(1,1-dimethylethyl)amino]carbonyl](3,3-dimethylpropyl)amino]-2-hydroxy-1-(phenylmethyl)propyl]-2-[(2-quinolinylcarbonyl)amino]-, [1S-[1R*(R*),2S*]]- | 3.7 |
| 6) | Propanamide, N-[3-[[[(1,1-dimethylethyl)amino]carbonyl] (3-methylbutyl)amino]-2-hydroxy-1-(phenylmethyl)propyl]-2-methyl-3-(methylsulfonyl)-, [1S-[1R*(R*), 2S*]]- | 29 |
| 7) | Propanamide, 3-(acetylamino)-N-[3-[[[(11,-dimethylethyl)amino]carbonyl] (3-methylbutyl)amino]-2-hydroxy-1-(phenylmethyl)propyl]-2-methyl-, [1S-[1R*(S*),2S*]]- | 100 |
| 8) | Propanamide, 3-(4-methoxybenzyloxycarbonyl)amino-N-[3-[[[(11,-dimethylethyl)amino]carbonyl](3-methylbutyl) amino]-2-hydroxy-1-(phenylmethyl) propyl]-2-methyl-, [1S-[1R*(S*),2S*]]- | 6 |
| 9) | Butanediamide, N¹-[2-hydroxy-3-[(4-fluorophenylmethyl)(1-pyrrolidinylcarbonyl)amino]-1-(phenylmethyl) propyl]-2-[(2-quinolinylcarbonyl) amino]-, [1S-[1R*(R*),2S*]]- | 7 |
| 10) | Butanediamide, N¹-[3-[[[(1,1-dimethylethyl)amino]carbonyl](3-methylbutyl)amino]-2-hydroxy-1-(phenylmethyl)propyl]-2,3,3-trimethyl-, [1S-[1R*(2S*),2S*]]- | 12 |

Utilizing the procedures set forth above in the examples along with the general description, it is contemplated that the compounds listed below could be prepared and that such compounds would have activities as HIV protease inhibitors substantially similar to the activities of the compounds set forth in the examples.
Propanamide, N-[3-[[[(1,1-dimethylethyl)amino]carbonyl]butylamino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](phenylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](4-fluorophenylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbohyl](4-methoxyphenylmethyl)amino]-2-hydroxy-1-(phenylmethyl)propyl]-2-methyl-3-(methylsulfonyl)-, [1S-[1R*(R*), 2S*]]-

The compounds of the present invention are effective antiviral compounds and, in particular, are effective retroviral inhibitors as shown above. Thus, the subject compounds are effective HIV protease inhibitors. It is contemplated that the subject compounds will also inhibit other viruses such as HIV, human T-cell leukemia virus, respiratory syncitial virus, hepadnavirus, cytomegalovirus and picornavirus.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate and undecanoate. Also, the basic nitrogen-containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more immunomodulators, antiviral agents or other antiinfective agents. When administered as a combination, the therapeutic agents can be formulated as separate compositions which are given at the same time or different times, or the therapeutic agents can be given as a single composition.

The foregoing is merely illustrative of the invention and is not intended to limit the invention to the disclosed compounds.

The preceding examples can be repeated with similar success by substituting the generically or specifically described reactants and/or operating conditions of this invention for those used in the preceding examples.

## Claims

1. Use of a compound represented by the formula: 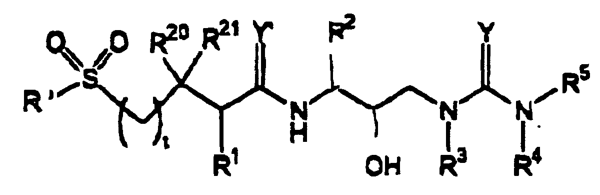 for preparing a medicament for inhibiting a retroviral protease wherein:
R' represents radicals as defined for R³;
R¹ represents hydrogen, -CH₂SO₂NH₂, alkyl and cycloalkyl radicals and amino acid side chains selected from asparagine, S-methyl cysteine and the corresponding sulfoxide and sulfone derivatives thereof, glycine, alloisoleucine, alanine, leucine, tert-leucine, phenylalanine, omithine, threonine, allo-threonine, isoleucine, histidine, norleucine, valine, serine, aspartic acid, beta-cyano alanine side chains;
R² represents alkyl, aryl, cycloalkyl, cycloalkylalkyl and aralkyl radicals optionally substituted with a group selected from -OR⁹, -SR⁹, and halogen radicals, wherein R⁹ represents hydrogen and alkyl radicals;
R³ represents alkyl, alkenyl, hydroxyalkyl, cycloalkyl, cycloalkylalkyl, heterocycloalkyl, heterocycloalkylalkyl, aryl, heteroaryl, aralkyl, and heteroaralkyl radicals;
R⁴ and R⁵, independently represent hydrogen and radicals as defined by R³, or R⁴ and R⁵ together with the nitrogen atom to which they are bonded represent heterocycloalkyl and heteroaryl radicals;
R²⁰ and R²¹ represnt radicals as defined for R¹; and
Y and Y' independently represent O and S
t represent 0 or 1; and
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom,
- heteroaryl is an aromatic monocyclic, bicyclic, or tricyclic heterocycle which contains the hetero atoms and is optionally substituted as defined above with respect to the definition of heterocycloalkyl,
- heteroaralkyl is a alkyl radical having 1 to 10 carbon atoms in which one hydrogen is replaced by a heteroaryl.

2. The use recited in Claim 1 wherein t is 0.

3. The use recited in Claim 1 wherein R¹ represents hydrogen and alkyl radicals; R²⁰ and R²¹ independently represent hydrogen and alkyl radicals; R' represents alkyl, aryl and aralkyl radicals; R³, R⁴ and R⁵ independently represent alkyl, alkenyl, hydroxyalkyl, cycloalkyl, cycloalkylalkyl, heterocycloalkyl, heterocycloalkylalkyl, aryl, aralkyl and heteroaralkyl radicals

4. The use recited in Claim 3 wherein R¹ represents alkyl radicals having from 1 to about 4 carbon atoms.

5. The use recited in Claim 4 wherein R¹ represents methyl, ethyl, isopropyl and t-butyl radicals.

6. The use recited in Claim 3 wherein R²⁰ and R²¹ independently represent hydrogen and methyl radicals.

7. The use recited in Claim 3 wherein R²⁰ is hydrogen and R²¹ is an alkyl radical.

8. The use recited in Claim 3 wherein R' is selected from methyl and phenethyl radicals.

9. The use recited in Claim 3 wherein R² represents alkyl, cycloalkylalkyl and aralkyl radicals.

10. The use recited in Claim 1 wherein R² represents CH₃SCH₂CH₂-, iso-butyl, n-butyl, benzyl, 4-f luorobenzyl, 2-naphthylmethyl and cyclohexylmethyl radicals.

11. The use recited in Claim 3 wherein R³ represents benzyl, para-fluorobenzyl, paramethoxybenzyl, para-methylbenzyl, and 2-naphthylmethyl radicals and R⁴ represents t-butyl.

12. The use recited in Claim 3 wherein R⁴ and R⁵ together with the nitrogen to which they are bonded represent pyrrolidinyl, piperidinyl, morpholinyl, and piperazinyl radicals.

13. The use recited in Claim 1 wherein the retroviral protease is HIV protease.

14. Use of a compound represented by the formula: 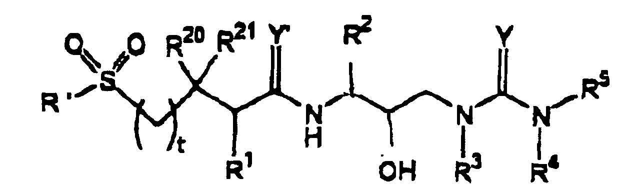 for preparing a medicament for treating a retroviral infection wherein:
R' represents radicals as defined for R³;
R¹ represents hydrogen -CH₂SO₂NH₂, alkyl and cycloalkyl radicals and amino acid side chains selected from asparagine, S-methyl cysteine and the corresponding sulfoxide and sulfone derivatives thereof, glycine, alloisoleucine, alanine, leucine, tert-leucine, phenylalanine, ornithine, threonine, allo-threonine, isoleucine, histidine, norleucine, valine, serine, aspartic acid, beta-cyano alanine side chains;
R² represents alkyl, aryl, cycloalkyl, cycloalkylalkyl and aralkyl radicals optionally substituted with a group selected from -OR⁹, -SR⁹, and halogen radicals, wherein R⁹ represents hydrogen and alkyl radicals;
R³ represents alkyl, alkenyl, hydroxyalkyl, cycloalkyl, cycloalkylalkyl, heterocycloalkyl, heterocycloalkylalkyl, aryl, heteroaryl, aralkyl, and heteroaralkyl radicals;
R⁴ and R⁵, independently represent hydrogen and radicals as defined by R³, or R⁴ and R⁵ together with the nitrogen atom to which they are bonded represent heterocycloalkyl and hetetoaryl radicals;
R²⁰ and R²¹ represent radicals as defined for R¹; and
Y and Y' independently represent O and S,
t represent 0 or 1; and
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom,
- heteroaryl is an aromatic monocyclic, bicyclic, or tricyclic heterocycle which contains the hetero atoms and is optionally substituted as defined above with respect to the definition of heterocycloalkyl,
- heteroaralkyl is a alkyl radical having 1 to 10 carbon atoms in which one hydrogen is replaced by a heteroaryl.

15. The use recited in Claim 14 wherein t is 0.

16. The use recited in Claim 14 wherein R¹ represents hydrogen and alkyl radicals; R²⁰ and R²¹ independently represent hydrogen and alkyl radicals; R' represents alkyl, aryl and aralkyl radicals; and R³, R⁴ and R⁵ independently represent alkyl, alkenyl, hydroxyalkyl, cycloalkyl, cycloalkylalkyl, heterocycloalkyl, heterocycloalkylalkyl, aryl, aralkyl and heteroaralkyl radicals.

17. The use recited in Claim 16 wherein R¹ represents alkyl radicals having from 1 to about 4 carbon atoms.

18. The use recited in Claim 17 wherein R¹ represents methyl, ethyl, isopropyl and t-butyl radicals.

19. The use recited in Claim 16 wherein R²⁰ and R²¹ independently represent hydrogen and methyl radicals.

20. The use recited in Claim 16 wherein R²⁰ is hydrogen and R²¹ is an alkyl radical.

21. The use recited in Claim 16 wherein R' is selected from methyl and phenethyl radicals.

22. The use recited in Claim 16 wherein R² represents alkyl, cycloalkylalkyl and aralkyl radicals.

23. The use recited in Claim 14 wherein R² represents CH₃SCH₂CH₂-, iso-butyl, n-butyl, benzyl, 4-f luorobenzyl, 2-naphthylmethyl and cyclohexylmethyl radicals.

24. The use recited in Claim 16 wherein R³ represents benzyl, para-fluorobenzyl, paramethoxybenzyl, para-methylbenzyl, and 2-naphthylmethyl radicals and R⁴ represents t-butyl.

25. The use recited in Claim 16 wherein R⁴ and R⁵ together with the nitrogen to which they are bonded represent pyrrolidinyl, piperidinyl, morpholinyl, and piperazinyl radicals.

26. The use recited in Claim 14 wherein the retroviral infection is an HIV infection.

27. Use of a compound represented by the formula : 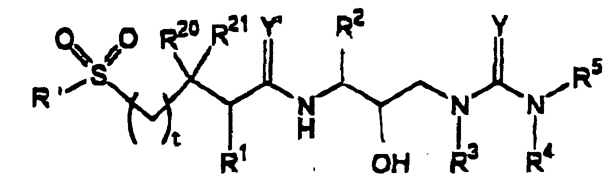 for preparing a medicament for for treating AIDS wherein:
R' represents radicals as defined for R³;
R¹ represents hydrogen -CH₂SO₂NH₂, alkyl and cycloalkyl radicals and amino acid side chains selected from asparagine, S-methyl cysteine and the corresponding sulfoxide and sulfone derivatives thereof, glycine, alloisoleucine, alanine, leucine, ten-leucine, phenylalanine, ornithine, threonine, allo-threonine, isoleucine, histidine, norleucine, valine, serine, aspartic acid, beta-cyano alanine side chains;
R² represents alkyl, aryl, cycloalkyl, cycloalkylalkyl and aralkyl radicals optionally substituted with a group selected from -OR⁹, -SR⁹, and halogen radicals, wherein R⁹ represents hydrogen and alkyl radicals;
R³ represents alkyl, alkenyl, hydroxyalkyl, cycloalkyl, cycloalkylatkyl, heterocycloalkyl, heterocycloalkylalkyl, aryl, heteroaryl, aralkyl, and heteroaralkyl radicals;
R⁴ and R⁵, independently represent hydrogen and radicals as defined by R³, or R⁴ and R⁵ together with the nitrogen atom to which they are banded represent heterocycloalkyl and heteroaryl radicals;
R²⁰ and R²¹ represent radicals as defined for R¹; and
Y and Y' independently represent O and S,
t represent 0 or 1; and
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro,
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom,
- heteroaryl is an aromatic monocyclic, bicyclic, or tricyclic heterocycle which contains the hetero atoms and is optionally substituted as defined above with respect to the definition of heterocycloalkyl,
- heteroaralkyl is a alkyl radical having 1 to 10 carbon atoms in which one hydrogen is replaced by a heteroaryl.

28. The use recited in Claim 27 wherein t is 0.

29. The use recited in Claim 27 wherein R¹ represents hydrogen and alkyl radicals; R²⁰ and R²¹ independently represent hydrogen and alkyl radicals; R' represents alkyl, aryl and aralkyl radicals; R³, R⁴ and R⁵ independently represent alkyl, alkenyl, hydroxyalkyl, cycloalkyl, cycloalkylalkyl, heterocycloalkyl, heterocycloalkylalkyl, aryl, aralkyl and heteroaralkyl radicals

30. The use recited in Claim 29 wherein R¹ represents alkyl radicals having from 1 to about 4 carbon atoms.

31. The use recited in Claim 30 wherein R¹ represents methyl, ethyl, isopropyl and t-butyl radicals.

32. The use recited in Claim 29 wherein R²⁰ and R²¹ independently represent hydrogen and methyl radicals.

33. The use recited in Claim 29 wherein R²⁰ is hydrogen and R²¹ is an alkyl radical.

34. The use recited in Claim 29 wherein R' is selected from methyl and phenethyl radicals.

35. The use recited in Claim 29 wherein R² represents alkyl, cycloalkylalkyl and aralkyl radicals.

36. The use recited in Claim 27 wherein R² represents CH₃SCH₂CH₂-, iso-butyl, n-butyl, benzyl, 4-fluorobenzyl, 2-naphthylmethyl and cyclohexylmethyl radicals.

37. The use recited in Claim 29 wherein R³ represents benzyl, para-fluorobenzyl, paramethoxybenzyl, para-methylbenzyl, and 2-naphthylmethyl radicals and R⁴ represents t-butyl.

38. The use recited in Claim 29 wherein R⁴ and R⁵ together with the nitrogen to which they are bonded represent pyrrolidinyl, piperidinyl, morpholinyl, and piperazinyl radicals.

39. The use recited in any of the claims 1, 14 and 27 wherein the compound is 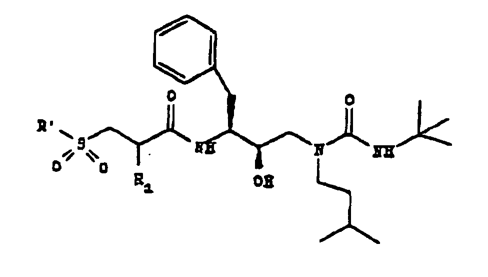 wherein R' is -CH₃ and R₁ is -CH(CH₃)₂.

40. The use recited in any one of the claims 1, 14 and 27 wherein the compound is selected among the group consisting of
Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)-, [1S-[1R*(R*),2S*]]-
Propanamide, N-[3-[[[(1,1-dimethylethyl)amino]carbonyl]butylamino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](phenylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](4-fluorophenylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](4-methoxyphenylmethyl)amino]-2-hydroxy-1-(phenylmethyl)propyl]-2-methyl-3-(methylsulfonyl)-, [1S-[1R*(R*), 2S*]]-

## Patentansprüche

1. Verwendung einer Verbindung mit der Formel 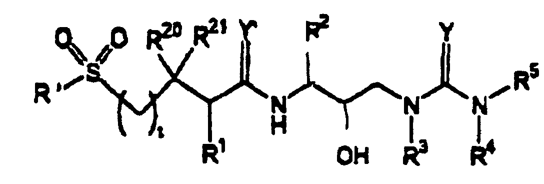 für die Herstellung eines Arzneimittels zum Inhibieren einer Retrovirusprotease, worin
- R' einen wie für R³ definierten Rest,
- R¹ Wasserstoff, -CH₂SO₂NH₂, einen Alkyl- und Cycloalkylrest und eine Aminosäureseitenkette, die aus Asparagin, S-Methylcystein und deren entsprechenden Sulfoxid- und Sulfonderivaten, einer Glycin-, Alloisoleucin-, Alanin-, Leucin-, *tert.*-Leucin-, Phenylalanin-, Ornithin-, Threonin-, Allothreonin-, Isoleucin-, Histidin-, Norleucin-, Valin-, Serin-, Asparaginsäure- und β-Cyanoalanin-Seitenkette ausgewählt ist,
- R² einen Alkyl-, Aryl-, Cycloalkyl-, Cycloalkylalkyl- und Aralkylrest, wahlweise substituiert mit einer Gruppe, die aus -OR⁹, -SR⁹ und Halogenresten ausgewählt ist, worin R⁹ Wasserstoff und einen Alkylrest bedeutet, und
- R³ einen Alkyl-, Alkenyl-, Hydroxyalkyl-, Cycloalkyl-, Cycloalkylalkyl-, Heterocycloalkyl-, Heterocycloalkylalkyl-, Aryl-, Heteroaryl-, Aralkyl- und Heteroaralkylrest bedeutet und
- R⁴ und R⁵ unabhängig voneinander Wasserstoff und einen wie für R³ definierten Rest bedeuten oder R⁴ und R⁵ zusammen mit dem Stickstoffatom, an welches sie gebunden sind, einen Heterocycloalkyl- und Heteroarylrest,
- R²⁰ und R²¹ jeweils einen wie für R¹ definierten Rest und
- Y und Y' unabhängig voneinander O und S bedeuten und
- t 0 oder 1 bedeutet, worin
• ein Alkylrest einen Alkylrest mit 1 bis 10 Kohlenstoffatomen,
• ein Cycloalkylrest einen Cycloalkylrest mit 3 bis 8 Kohlenstoffatomen,
• ein Aralkylrest einen Alkylrest mit 1 bis 10 Kohlenstoffatomen, worin ein Wasserstoffatom durch einen Arylrest ersetzt ist,
• ein Arylrest einen Phenyl- oder Naphthylrest, der wahlweise einen oder mehrere Substituenten trägt, die aus Alkyl, Alkoxy, Halogen, Hydroxy, Amino und Nitro ausgewählt sind,
• ein Heterocycloalkylrest einen gesättigten oder teilweise ungesättigten monocyclischen, bicyclischen oder tricyclischen Heterocyclus, der ein oder mehrere Heteroatome enthält, die aus Stickstoff, Sauerstoff und Schwefel ausgewählt sind, und welcher wahlweise an einem oder mehreren Kohlenstoffatomen durch Halogen, Alkyl, Alkoxy oder Oxo und/oder an einem sekundären Stickstoffatom durch Alkyl, Aralkoxycarbonyl, Alkanoyl, Phenyl oder Phenylalkyl oder an einem tertiären Stickstoffatom durch Oxido, das über ein Kohlenstoffatom befestigt ist, substituiert ist,
• ein Heteroarylrest einen aromatischen monocyclischen, bicyclischen oder tricyclischen Heterocyclus, der die Heteroatome enthält und wahlweise wie zuvor hinsichtlich dem Heterocycloalkyl definiert substituiert ist, und
• ein Heteroaralkylrest einen Alkylrest mit 1 bis 10 Kohlenstoffatomen, in welchem ein Wasserstoff durch ein Heteroaryl ersetzt ist
bedeutet.

2. Verwendung nach Anspruch 1, worin t 0 bedeutet.

3. Verwendung nach Anspruch 1, worin R' Wasserstoff und einen Alkylrest bedeutet, R²⁰ und R²¹ unabhängig voneinander Wasserstoff und einen Alkylrest bedeuten, R' einen Alkyl-, Aryl- und Aralkylrest bedeutet und R³, R⁴ und R⁵ unabhängig voneinander einen Alkyl-, Alkenyl-, Hydroxyalkyl-, Cycloalkyl-, Cycloalkylalkyl-, Heterocycloalkyl, Heterocycloalkylalkyl-, Aryl-, Aralkyl- und Heteroaralkylrest bedeuten.

4. Verwendung nach Anspruch 3, worin R¹ einen Alkylrest mit 1 bis etwa 4 Kohlenstoffatomen bedeutet.

5. Verwendung nach Anspruch 4, worin R¹ einen Methyl-, Ethyl-, Isopropyl- und tert.-Butylrest bedeutet.

6. Verwendung nach Anspruch 3, worin R²⁰ und R²¹ unabhängig voneinander einen Wasserstoff- und einen Methylrest bedeuten.

7. Verwendung nach Anspruch 3, worin R²⁰ Wasserstoff und R²¹ einen Alkylrest bedeutet.

8. Verwendung nach Anspruch 3, worin R' aus einem Methyl- und Phenethylrest ausgewählt ist.

9. Verwendung nach Anspruch 3, worin R² einen Alkyl-, Cycloalkylalkyl- und Aralkylrest bedeutet.

10. Verwendung nach Anspruch 1, worin R² CH₃SCH₂CH₂-, einen Isobutyl-, n-Butyl-, Benzyl-, 4-Fluorbenzyl-, 2-Naphthylmethyl- und Cyclohexylmethylrest bedeutet.

11. Verwendung nach Anspruch 3, worin R³ einen Benzyl-, p-Fluorbenzyl-, p-Methoxybenzyl-, p-Methylbenzyl und 2-Naphthylmethylrest und R⁴ einen *tert.*-Butylrest bedeutet.

12. Verwendung nach Anspruch 3, worin R⁴ und R⁵ zusammen mit dem Stickstoff, an welchen sie gebunden sind, einen Pyrrolidinyl-, Piperidinyl-, Morpholinyl- und Piperazinylrest bedeuten.

13. Verwendung nach Anspruch 1, worin die Retrovirusprotease HIV-Protease ist.

14. Verwendung einer Verbindung mit der Formel 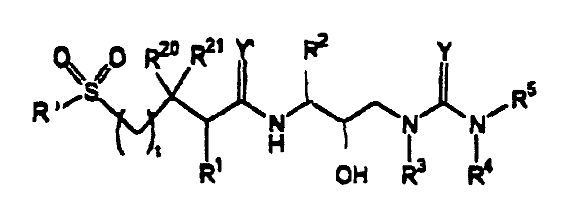 für die Herstellung eines Arzneimittels zur Behandlung einer Retrovirusinfektion, worin:
- R' einen wie für R³ definierten Rest,
- R¹ Wasserstoff, -CH₂SO₂NH₂, einen Alkyl- und Cycloalkylrest und eine Aminosäureseitenkette, die aus Asparagin, S-Methylcystein und deren entsprechenden Sulfoxid- und Sulfonderivaten, einer Glycin-, Alloisoleucin-, Alanin-, Leucin-, tert.-Leucin-, Phenylalanin-, Ornithin-, Threonin-, Allothreonin-, Isoleucin-, Histidin-, Norleucin-, Valin-, Serin-, Asparaginsäure- und β-Cyanoalanin-Seitenkette ausgewählt ist,
- R² einen Alkyl-, Aryl-, Cycloalkyl-, Cycloalkylalkyl- und Aralkylrest, wahlweise substituiert mit einer Gruppe, die aus -OR⁹, -SR⁹ und Halogenresten ausgewählt ist, worin R⁹ Wasserstoff und einen Alkylrest bedeutet, und
- R³ einen Alkyl-, Alkenyl-, Hydroxyalkyl-, Cycloalkyl-, Cycloalkylalkyl-, Heterocycloalkyl-, Heterocycloalkylalkyl-, Aryl-, Heteroaryl-, Aralkyl- und Heteroaralkylrest bedeutet und
- R⁴ und R⁵ unabhängig voneinander Wasserstoff und einen wie für R³ definierten Rest bedeuten oder R⁴ und R⁵ zusammen mit dem Stickstoffatom, an welches sie gebunden sind, einen Heterocycloalkyl- und Heteroarylrest,
- R²⁰ und R²¹ jeweils einen wie für R¹ definierten Rest und
- Y und Y' unabhängig voneinander O und S bedeuten und
- t 0 oder 1 bedeutet, worin
• ein Alkylrest einen Alkylrest mit 1 bis 10 Kohlenstoffatomen,
• ein Cycloalkylrest einen Cycloalkylrest mit 3 bis 8 Kohlenstoffatomen,
• ein Aralkylrest einen Alkylrest mit 1 bis 10 Kohlenstoffatomen, worin ein Wasserstoffatom durch einen Arylrest ersetzt ist,
• ein Arylrest einen Phenyl- oder Naphthylrest, der wahlweise einen oder mehrere Substituenten trägt, die aus Alkyl, Alkoxy, Halogen, Hydroxy, Amino und Nitro ausgewählt sind,
• ein Heterocycloalkylrest einen gesättigten oder teilweise ungesättigten monocyclischen, bicyclischen oder tricyclischen Heterocyclus, der ein oder mehrere Heteroatome enthält, die aus Stickstoff, Sauerstoff und Schwefel ausgewählt sind, und welcher wahlweise an einem oder mehreren Kohlenstoffatomen durch Halogen, Alkyl, Alkoxy oder Oxo und/oder an einem sekundären Stickstoffatom durch Alkyl, Aralkoxycarbonyl, Alkanoyl, Phenyl oder Phenylalkyl oder an einem tertiären Stickstoffatom durch Oxido, das über ein Kohlenstoffatom befestigt ist, substituiert ist,
• ein Heteroarylrest einen aromatischen monocyclischen, bicyclischen oder tricyclischen Heterocyclus, der die Heteroatome enthält und wahlweise wie zuvor hinsichtlich dem Heterocycloalkyl definiert substituiert ist, und
• ein Heteroaralkylrest einen Alkylrest mit 1 bis 10 Kohlenstoffatomen, in welchem ein Wasserstoff durch ein Heteroaryl ersetzt ist
bedeutet.

15. Verwendung nach Anspruch 14, worin t 0 bedeutet.

16. Verwendung nach Anspruch 14, worin R¹ Wasserstoff und einen Alkylrest bedeutet, R²⁰ und R²¹ unabhängig voneinander Wasserstoff und einen Alkylrest bedeuten, R' einen Alkyl-, Aryl- und Aralkylrest bedeutet und R³, R⁴ und R⁵ unabhängig voneinander einen Alkyl-, Alkenyl-, Hydroxyalkyl-, Cycloalkyl-, Cycloalkylalkyl-, Heterocycloalkyl, Heterocycloalkylalkyl-, Aryl-, Aralkyl- und Heteroaralkylrest bedeuten.

17. Verwendung nach Anspruch 16, worin R¹ einen Alkylrest mit 1 bis etwa 4 Kohlenstoffatomen bedeutet.

18. Verwendung nach Anspruch 17, worin R¹ einen Methyl-, Ethyl-, Isopropyl- und tert.-Butylrest bedeutet.

19. Verwendung nach Anspruch 16, worin R²⁰ und R²¹ unabhängig voneinander einen Wasserstoff- und einen Methylrest bedeuten.

20. Verwendung nach Anspruch 16, worin R²⁰ Wasserstoff und R²¹ einen Alkylrest bedeutet.

21. Verwendung nach Anspruch 16, worin R' aus einem Methyl- und Phenethylrest ausgewählt ist.

22. Verwendung nach Anspruch 16, worin R² einen Alkyl-, Cycloalkylalkyl- und Aralkylrest bedeutet.

23. Verwendung nach Anspruch 14, worin R² CH₃SCH₂CH₂-, einen Isobutyl-, n-Butyl-, Benzyl-, 4-Fluorbenzyl-, 2-Naphthylmethyl- und Cyclohexylmethylrest bedeutet.

24. Verwendung nach Anspruch 16, worin R³ einen Benzyl-, p-Fluorbenzyl-, p-Methoxybenzyl-, p-Methylbenzyl und 2-Naphthylmethylrest und R⁴ einen *tert*.-Butylrest bedeutet.

25. Verwendung nach Anspruch 16, worin R⁴ und R⁵ zusammen mit dem Stickstoff, an welchen sie gebunden sind, einen Pyrrolidinyl-, Piperidinyl-, Morpholinyl- und Piperazinylrest bedeuten.

26. Verwendung nach Anspruch 14, wobei die Retrovirusinfektion eine HIV-Infektion ist.

27. Verwendung einer Verbindung mit der Formel 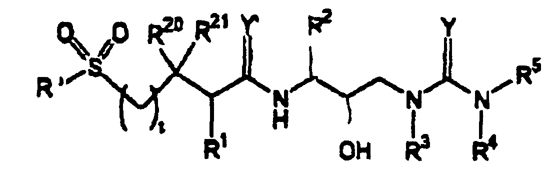 für die Herstellung eines Arzneimittels zur Behandlung von AIDS, worin:
- R' einen wie für R³ definierten Rest,
- R¹ Wasserstoff, -CH₂SO₂NH₂, einen Alkyl- und Cycloalkylrest und eine Aminosäureseitenkette, die aus Asparagin, S-Methylcystein und deren entsprechenden Sulfoxid- und Sulfonderivaten, einer Glycin-, Alloisoleucin-, Alanin-, Leucin-, *tert*.-Leucin-, Phenylalanin-, Ornithin-, Threonin-, Allothreonin-, Isoleucin-, Histidin-, Norleucin-, Valin-, Serin-, Asparaginsäure- und β-Cyanoalanin-Seitenkette ausgewählt ist,
- R² einen Alkyl-, Aryl-, Cycloalkyl-, Cycloalkylalkyl- und Aralkylrest, wahlweise substituiert mit einer Gruppe, die aus -OR⁹, -SR⁹ und Halogenresten ausgewählt ist, worin R⁹ Wasserstoff und einen Alkylrest bedeutet, und
- R³ einen Alkyl-, Alkenyl-, Hydroxyalkyl-, Cycloalkyl-, Cycloalkylalkyl-, Heterocycloalkyl-, Heterocycloalkylalkyl-, Aryl-, Heteroaryl-, Aralkyl- und Heteroaralkylrest bedeutet und
- R⁴ und R⁵ unabhängig voneinander Wasserstoff und einen wie für R³ definierten Rest bedeuten oder R⁴ und R⁵ zusammen mit dem Stickstoffatom, an welches sie gebunden sind, einen Heterocycloalkyl- und Heteroarylrest,
- R²⁰ und R²¹ jeweils einen wie für R¹ definierten Rest und
- Y und Y' unabhängig voneinander O und S bedeuten und
- t 0 oder 1 bedeutet, worin
• ein Alkylrest einen Alkylrest mit 1 bis 10 Kohlenstoffatomen,
• ein Cycloalkylrest einen Cycloalkylrest mit 3 bis 8 Kohlenstoffatomen,
• ein Aralkylrest einen Alkylrest mit 1 bis 10 Kohlenstoffatomen, worin ein Wasserstoffatom durch einen Arylrest ersetzt ist,
• ein Arylrest einen Phenyl- oder Naphthylrest, der wahlweise einen oder mehrere Substituenten trägt, die aus Alkyl, Alkoxy, Halogen, Hydroxy, Amino und Nitro ausgewählt sind,
• ein Heterocycloalkylrest einen gesättigten oder teilweise ungesättigten monocyclischen, bicyclischen oder tricyclischen Heterocyclus, der ein oder mehrere Heteroatome enthält, die aus Stickstoff, Sauerstoff und Schwefel ausgewählt sind, und welcher wahlweise an einem oder mehreren Kohlenstoffatomen durch Halogen, Alkyl, Alkoxy oder Oxo und/oder an einem sekundären Stickstoffatom durch Alkyl, Aralkoxycarbonyl, Alkanoyl, Phenyl oder Phenylalkyl oder an einem tertiären Stickstoffatom durch Oxido, das über ein Kohlenstoffatom befestigt ist, substituiert ist,
• ein Heteroarylrest einen aromatischen monocyclischen, bicyclischen oder tricyclischen Heterocyclus, der die Heteroatome enthält und wahlweise wie zuvor hinsichtlich dem Heterocycloalkyl definiert substituiert ist, und
• ein Heteroaralkylrest einen Alkylrest mit 1 bis 10 Kohlenstoffatomen, in welchem ein Wasserstoff durch ein Heteroaryl ersetzt ist
bedeutet.

28. Verwendung nach Anspruch 27, worin t 0 bedeutet.

29. Verwendung nach Anspruch 27, worin R¹ Wasserstoff und einen Alkylrest bedeutet, R²⁰ und R²¹ unabhängig voneinander Wasserstoff und einen Alkylrest bedeuten, R' einen Alkyl-, Aryl- und Aralkylrest bedeutet und R³, R⁴ und R⁵ unabhängig voneinander einen Alkyl-, Alkenyl-, Hydroxyalkyl-, Cycloalkyl-, Cycloalkylalkyl-, Heterocycloalkyl, Heterocycloalkylalkyl-, Aryl-, Aralkyl- und Heteroaralkylrest bedeuten.

30. Verwendung nach Anspruch 29, worin R¹ einen Alkylrest mit 1 bis etwa 4 Kohlenstoffatomen bedeutet.

31. Verwendung nach Anspruch 30, worin R¹ einen Methyl-, Ethyl-, Isopropyl- und tert.-Butylrest bedeutet.

32. Verwendung nach Anspruch 29, worin R²⁰ und R²¹ unabhängig voneinander einen Wasserstoff- und einen Methylrest bedeuten.

33. Verwendung nach Anspruch 29, worin R²⁰ Wasserstoff und R²¹ einen Alkylrest bedeutet.

34. Verwendung nach Anspruch 29, worin R' aus einem Methyl- und Phenethylrest ausgewählt ist.

35. Verwendung nach Anspruch 29, worin R² einen Alkyl-, Cycloalkylalkyl- und Aralkylrest bedeutet.

36. Verwendung nach Anspruch 27, worin R² CH₃SCH₂CH₂-, einen Isobutyl-, n-Butyl-, Benzyl-, 4-Fluorbenzyl-, 2-Naphthylmethyl- und Cyclohexylmethylrest bedeutet.

37. Verwendung nach Anspruch 29, worin R³ einen Benzyl-, p-Fluorbenzyl-, p-Methoxybenzyl-, p-Methylbenzyl und 2-Naphthylmethylrest und R⁴ einen *tert*.-Butylrest bedeutet.

38. Verwendung nach Anspruch 29, worin R⁴ und R⁵ zusammen mit dem Stickstoff, an welchen sie gebunden sind, einen Pyrrolidinyl-, Piperidinyl-, Morpholinyl- und Piperazinylrest bedeuten.

39. Verwendung nach einem der Ansprüche 1, 14 und 27, wobei die Verbindung 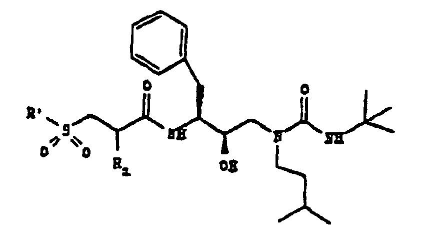 ist, worin R' -CH₃ und R₁ -CH(CH₃)₂ bedeutet.

40. Verwendung nach einem der Ansprüche 1, 14 und 27, wobei die Verbindung aus der Gruppe ausgewählt ist, die besteht aus
[1S-[1R*(R*),2S*]]-N-[3-[[[1,1-Dimethylethyl)amino]carbonyl](3-methylbutyl)amino]-2-hydroxy-1-(phenylmethyl)propyl]-2-methyl-3-(methylsulfonyl)-propanamid,
[1S-[1R*(R*),2S*]]-N-[3-[[[(1,1-Dimethylethyl)amino]carbonyl]butylamino]-2-hydroxy-1-(phenylmethyl)propyl]-2-methyl-3-(methylsulfonyl)-propanamid,
[1S-[1R-(R*),2S*]]-N-[3-[[[(1,1-Dimethylethyl)aminocarbonyl](phenylmethyl)amino]-2-hydroxy-1-(phenylmethyl)propyl]-2-methyl-3-(methylsulfonyl)-propanamid,
[1S-[1R*(R*),2S*]]-N-[3-[[[(1,1-Dimethylethyl)aminocarbonyl](4-fluorphenylmethyl)amino]-2-hydroxy-1-(phenylmethyl)propyl]-2-methyl-3-(methylsulfonyl)-propanamid,
[1S-[1R*(R*),2S*]]-N-[3-[[[(1,1-Dimethylethyl)aminocarbonyl](4-methoxyphenylmethyl)amino]-2-hydroxy-1-(phenylmethyl)propyl]-2-methyl-3-(methylsulfonyl)-propanamid.

## Revendications

1. Utilisation d'un composé représenté par la formule : 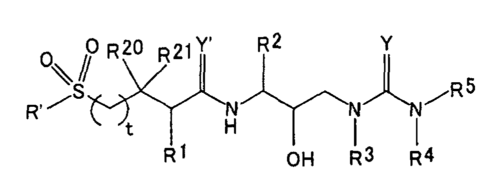 pour préparer un médicament pour inhiber une protéase rétrovirale, dans laquelle :
R' représente des radicaux tels que définis pour R³ ;
R¹ représente hydrogène, -CH₂SO₂NH₂, des radicaux alkyle et cycloalkyle et des chaînes latérales d'acide aminé, choisi parmi l'asparagine, la S-méthylcystéine et ses dérivés sulfoxyde et sulfone correspondants, la glycine, l'alloisoleucine, l'alanine, la leucine, la t-leucine, la phénylalanine, l'ornithine, la thréonine, l'allothréonine, l'isoleucine, l'histidine, la norleucine, la valine, la sérine, l'acide aspartique, la bêta-cyanoalanine ;
R² représente des radicaux alkyle, aryle, cycloalkyle, cycloalkylalkyle et aralkyle, le cas échéant substitués par un radical choisi parmi -OR⁹, -SR⁹, et des radicaux halogène, où R⁹ représente hydrogène et des radicaux alkyle ;
R³ représente des radicaux alkyle, alcényle, hydroxyalkyle, cycloalkyle, cycloalkylalkyle, hétérocycloalkyle, hétérocycloalkylalkyle, aryle, hétéroaryle, aralkyle et hétéroaralkyle ;
R⁴ et R⁵ représentent indépendamment, hydrogène et les radicaux tels que définis pour R³, ou R⁴ et R⁵ représentent avec l'atome d'azote auquel ils sont liés, des radicaux hétérocycloalkyle et hétéroaryle ;
R²⁰ et R²¹ représentent les radicaux tels que définis pour R¹, et
Y et Y' représentent indépendamment, O et S,
t représente 0 ou 1, et
dans laquelle :
- alkyle signifie un alkyle ayant 1 à 10 atomes de carbone,
- cycloalkyle signifie un cycloalkyle ayant 3 à 8 atomes de carbone,
- aralkyle signifie un alkyle ayant 1 à 10 atomes de carbone, dans lequel un hydrogène est remplacé par un aryle,
- aryle est phényle ou naphtyle, qui porte le cas échéant, un ou plusieurs substituants choisis parmi alkyle, alcoxy, halogène, hydroxy, amino et nitro,
- hétérocycloalkyle est un hétérocycle saturé ou partiellement insaturé, monocyclique, bicyclique ou tricyclique, qui contient un ou plusieurs hétéroatomes choisis parmi azote, oxygène et soufre, qui est le cas échéant substitué sur un ou plusieurs atomes de carbone, par halogène, alkyle, alcoxy ou oxo, et/ou sur un atome d'azote secondaire, par alkyle, aralcoxycarbonyle, alcanoyle, phényle ou phénylalkyle ou sur un atome d'azote tertiaire, par oxydo et qui est attaché via un atome de carbone,
- hétéroaryle est un hétérocycle aromatique monocyclique, bicyclique ou tricyclique, qui contient les hétéroatomes et qui est le cas échéant, substitué tel que défini ci-dessus pour la définition de hétérocycloalkyle,
- hétéroaralkyle est un radical alkyle ayant 1 à 10 atomes de carbone, dans lequel un hydrogène est remplacé par un hétéroaryle.

2. Utilisation selon la revendication 1, où t est 0.

3. Utilisation selon la revendication 1, où R¹ représente hydrogène et des radicaux alkyle ; R²⁰ et R²¹ représentent indépendamment, hydrogène et des radicaux alkyle ; R' représente des radicaux alkyle, aryle et aralkyle ; R³, R⁴ et R⁵ représentent indépendamment, des radicaux alkyle, alcényle, hydroxyalkyle, cycloalkyle, cycloalkylalkyle, hétérocycloalkyle, hétérocycloalkylalkyle, aryle, aralkyle et hétéroaralkyle.

4. Utilisation selon la revendication 3, où R¹ représente des radicaux alkyle ayant 1 à environ 4 atomes de carbone.

5. Utilisation selon la revendication 4, où R¹ représente les radicaux méthyle, éthyle, isopropyle et t-butyle.

6. Utilisation selon la revendication 3, où R²⁰ et R²¹ représentent indépendamment, hydrogène et le radical méthyle.

7. Utilisation selon la revendication 3, où R²⁰ est hydrogène et R²¹ est un radical alkyle.

8. Utilisation selon la revendication 3, où R' est choisi parmi les radicaux méthyle et phénéthyle.

9. Utilisation selon la revendication 3, où R⁹ représente les radicaux alkyle, cycloalkyle et aralkyle.

10. Utilisation selon la revendication 1, où R² représente les radicaux CH₃SCH₂CH₂-, isobutyle, n-butyle, benzyle, 4-fluorobenzyle, 2-naphtylméthyle et cyclohexylméthyle.

11. Utilisation selon la revendication 3, où R³ représente les radicaux benzyle, para-fluorobenzyle, para-méthoxybenzyle, para-méthylbenzyle et 2-naphtylméthyle et R⁴ représente t-butyle.

12. Utilisation selon la revendication 3, où R⁴ et R⁵ représentent avec l'azote auquel ils sont liés, les radicaux pyrrolidinyle, pipéridinyle, morpholinyle et pipérazinyle.

13. Utilisation selon la revendication 1, où la protéase rétrovirale est la protéase de HIV.

14. Utilisation d'un composé représenté par la formule : 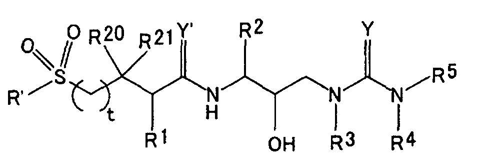 pour préparer un médicament pour traiter une infection rétrovirale, dans laquelle :
R' représente des radicaux tels que définis pour R³ ;
R¹ représente hydrogène, -CH₂SO₂NH₂, des radicaux alkyle et cycloalkyle et des chaînes latérales d'acide aminé, choisi parmi l'asparagine, la S-méthylcystéine et ses dérivés sulfoxyde et sulfone correspondants, la glycine, l'alloisoleucine, l'alanine, la leucine, la t-leucine, la phénylalanine, l'ornithine, la thréonine, l'allothréonine, l'isoleucine, l'histidine, la norleucine, la valine, la sérine, l'acide aspartique, la bêta-cyanoalanine ;
R² représente des radicaux alkyle, aryle, cycloalkyle, cycloalkylalkyle et aralkyle, le cas échéant substitués par un radical choisi parmi -OR⁹, -SR⁹, et des radicaux halogène, où R⁹ représente hydrogène et des radicaux alkyle ;
R³ représente des radicaux alkyle, alcényle, hydroxyalkyle, cycloalkyle, cycloalkylalkyle, hétérocycloalkyle, hétérocycloalkylalkyle, aryle, hétéroaryle, aralkyle et hétéroaralkyle ;
R⁴ et R⁵ représentent indépendamment, hydrogène et les radicaux tels que définis pour R³, ou R⁴ et R⁵ représentent avec l'atome d'azote auquel ils sont liés, des radicaux hétérocycloalkyle et hétéroaryle ;
R²⁰ et R²¹ représentent les radicaux tels que définis pour R¹, et
Y et Y' représentent indépendamment, O et S,
t représente 0 ou 1, et
dans laquelle :
- alkyle signifie un alkyle ayant 1 à 10 atomes de carbone,
- cycloalkyle signifie un cycloalkyle ayant 3 à 8 atomes de carbone,
- aralkyle signifie un alkyle ayant 1 à 10 atomes de carbone, dans lequel un hydrogène est remplacé par un aryle,
- aryle est phényle ou naphtyle, qui porte le cas échéant, un ou plusieurs substituants choisis parmi alkyle, alcoxy, halogène, hydroxy, amino et nitro,
- hétérocycloalkyle est un hétérocycle saturé ou partiellement insaturé, monocyclique, bicyclique ou tricyclique, qui contient un ou plusieurs hétéroatomes choisis parmi azote, oxygène et soufre, qui est le cas échéant substitué sur un ou plusieurs atomes de carbone, par halogène, alkyle, alcoxy ou oxo, et/ou sur un atome d'azote secondaire, par alkyle, aralcoxycarbonyle, alcanoyle, phényle ou phénylalkyle ou sur un atome d'azote tertiaire, par oxydo et qui est attaché via un atome de carbone,
- hétéroaryle est un hétérocycle aromatique monocyclique, bicyclique ou tricyclique, qui contient les hétéroatomes et qui est le cas échéant, substitué tel que défini ci-dessus pour la définition de hétérocycloalkyle,
- hétéroaralkyle est un radical alkyle ayant 1 à 10 atomes de carbone, dans lequel un hydrogène est remplacé par un hétéroaryle.

15. Utilisation selon la revendication 14, où t est 0.

16. Utilisation selon la revendication 14, où R¹ représente hydrogène et des radicaux alkyle ; R²⁰ et R²¹ représentent indépendamment, hydrogène et des radicaux alkyle ; R' représente des radicaux alkyle, aryle et aralkyle ; R³, R⁴ et R⁵ représentent indépendamment, des radicaux alkyle, alcényle, hydroxyalkyle, cycloalkyle, cycloalkylalkyle, hétérocycloalkyle, hétérocycloalkylalkyle, aryle, aralkyle et hétéroaralkyle.

17. Utilisation selon la revendication 16, où R¹ représente des radicaux alkyle ayant 1 à environ 4 atomes de carbone.

18. Utilisation selon la revendication 17, où R¹ représente les radicaux méthyle, éthyle, isopropyle et t-butyle.

19. Utilisation selon la revendication 16, où R²⁰ et R²¹ représentent indépendamment, hydrogène et le radical méthyle.

20. Utilisation selon la revendication 16, où R²⁰ est hydrogène et R²¹ est un radical alkyle.

21. Utilisation selon la revendication 16, où R' est choisi parmi les radicaux méthyle et phénéthyle.

22. Utilisation selon la revendication 16, où R² représente les radicaux alkyle, cycloalkyle et aralkyle.

23. Utilisation selon la revendication 14, où R² représente les radicaux CH₃SCH₂CH₂-, isobutyle, n-butyle, benzyle, 4-fluorobenzyle, 2-naphtylméthyle et cyclohexylméthyle.

24. Utilisation selon la revendication 16, où R³ représente les radicaux benzyle, para-fluorobenzyle, para-méthoxybenzyle, para-méthylbenzyle et 2-naphtylméthyle et R⁴ représente t-butyle.

25. Utilisation selon la revendication 16, où R⁴ et R⁵ représentent avec l'azote auquel ils sont liés, les radicaux pyrrolidinyle, pipéridinyle, morpholinyle et pipérazinyle.

26. Utilisation selon la revendication 14, où l'infection protéase rétrovirale est une infection HIV.

27. Utilisation d'un composé représenté par la formule : 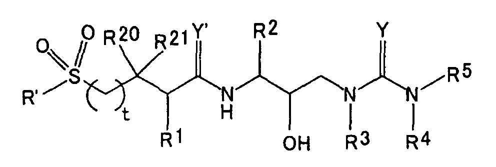 pour préparer un médicament pour traiter le SIDA, dans laquelle :
R' représente des radicaux tels que définis pour R³;
R¹ représente hydrogène, -CH₂SO₂NH₂, des radicaux alkyle et cycloalkyle et des chaînes latérales d'acide aminé, choisi parmi l'asparagine, la S-méthylcystéine et ses dérivés sulfoxyde et sulfone correspondants, la glycine, l'alloisoleucine, l'alanine, la leucine, la t-leucine, la phénylalanine, l'ornithine, la thréonine, l'allothréonine, l'isoleucine, l'histidine, la norleucine, la valine, la sérine, l'acide aspartique, la bêta-cyanoalanine ;
R² représente des radicaux alkyle, aryle, cycloalkyle, cycloalkylalkyle et aralkyle, le cas échéant substitués par un radical choisi parmi -OR⁹, -SR⁹, et des radicaux halogène, où R⁹ représente hydrogène et des radicaux alkyle ;
R³ représente des radicaux alkyle, alcényle, hydroxyalkyle, cycloalkyle, cycloalkylalkyle, hétérocycloalkyle, hétérocycloalkylalkyle, aryle, hétéroaryle, aralkyle et hétéroaralkyle ;
R⁴ et R⁵ représentent indépendamment, hydrogène et les radicaux tels que définis pour R³, ou R⁴ et R⁵ représentent avec l'atome d'azote auquel ils sont liés, des radicaux hétérocycloalkyle et hétéroaryle ;
R²⁰ et R²¹ représentent les radicaux tels que définis pour R¹, et
Y et Y' représentent indépendamment, O et S,
t représente 0 ou 1, et
dans laquelle :
- alkyle signifie un alkyle ayant 1 à 10 atomes de carbone,
- cycloalkyle signifie un cycloalkyle ayant 3 à 8 atomes de carbone,
- aralkyle signifie un alkyle ayant 1 à 10 atomes de carbone, dans lequel un hydrogène est remplacé par un aryle,
- aryle est phényle ou naphtyle, qui porte le cas échéant, un ou plusieurs substituants choisis parmi alkyle, alcoxy, halogène, hydroxy, amino et nitro,
- hétérocycloalkyle est un hétérocycle saturé ou partiellement insaturé, monocyclique, bicyclique ou tricyclique, qui contient un ou plusieurs hétéroatomes choisis parmi azote, oxygène et soufre, qui est le cas échéant substitué sur un ou plusieurs atomes de carbone, par halogène, alkyle, alcoxy ou oxo, et/ou sur un atome d'azote secondaire, par alkyle, aralcoxycarbonyle, alcanoyle, phényle ou phénylalkyle ou sur un atome d'azote tertiaire, par oxydo et qui est attaché via un atome de carbone,
- hétéroaryle est un hétérocycle aromatique monocyclique, bicyclique ou tricyclique, qui contient les hétéroatomes et qui est le cas échéant, substitué tel que défini ci-dessus pour la définition de hétérocycloalkyle,
- hétéroaralkyle est un radical alkyle ayant 1 à 10 atomes de carbone, dans lequel un hydrogène est remplacé par un hétéroaryle.

28. Utilisation selon la revendication 27, où t est 0.

29. Utilisation selon la revendication 27, où R¹ représente hydrogène et des radicaux alkyle ; R²⁰ et R²¹ représentent indépendamment, hydrogène et des radicaux alkyle ; R' représente des radicaux alkyle, aryle et aralkyle ; R³, R⁴ et R⁵ représentent indépendamment, des radicaux alkyle, alcényle, hydroxyalkyle, cycloalkyle, cycloalkylalkyle, hétérocycloalkyle, hétérocycloalkylalkyle, aryle, aralkyle et hétéroaralkyle.

30. Utilisation selon la revendication 29, où R¹ représente des radicaux alkyle ayant 1 à environ 4 atomes de carbone.

31. Utilisation selon la revendication 30, où R¹ représente les radicaux méthyle, éthyle, isopropyle et t-butyle.

32. Utilisation selon la revendication 29, où R²⁰ et R²¹ représentent indépendamment, hydrogène et le radical méthyle.

33. Utilisation selon la revendication 29, où R²⁰ est hydrogène et R²¹ est un radical alkyle.

34. Utilisation selon la revendication 29, où R' est choisi parmi les radicaux méthyle et phénéthyle.

35. Utilisation selon la revendication 29, où R² représente les radicaux alkyle, cycloalkyle et aralkyle.

36. Utilisation selon la revendication 27, où R² représente les radicaux CH₃SCH₂CH₂-, isobutyle, n-butyle, benzyle, 4-fluorobenzyle, 2-naphtylméthyle et cyclohexylméthyle.

37. Utilisation selon la revendication 29, où R³ représente les radicaux benzyle, para-fluorobenzyle, para-méthoxybenzyle, para-méthylbenzyle et 2-naphtylméthyle et R⁴ représente t-butyle.

38. Utilisation selon la revendication 29, où R⁴ et R⁵ représentent avec l'azote auquel ils sont liés, les radicaux pyrrolidinyle, pipéridinyle, morpholinyle et pipérazinyle.

39. Utilisation selon la revendication 1, 14 et 27, où le composé est : 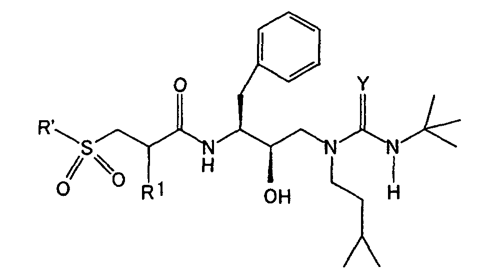 où R' est CH₃ et R¹ est CH(CH₃)₂.

40. Utilisation selon la revendication 1, 14 et 27, où le composé est choisi parmi le groupe consistant en :
le propanamide, N-[3-[[[(1,1-diméthyléthyl)-amino]carbonyl]butylamino]-2-hydroxy-1-(phénylméthyl)-propyl]-2-méthyl-3-(méthylsulfonyl)-, [1S-[1R*(R*),2S*]]-
le propanamide, N-[3-[[[(1,1-diméthyléthyl)-amino]carbonyl](phénylméthyl)amino]-2-hydroxy-1-(phénylméthyl)propyl]-2-méthyl-3-(méthylsulfonyl)-, [1S-[1R*(R*),2S*]]-
le propanamide, N-[3-[[[(1,1-diméthyléthyl)-amino]carbonyl](4-fluorophénylméthyl)amino]-2-hydroxy-1-(phénylméthyl)propyl]-2-méthyl-3-(méthylsulfonyl)-, [1S-[1R*(R*),2S*]]-
le propanamide, N-[3-[[[(1,1-diméthyléthyl)-amino]carbonyl](4-méthoxyphénylméthyl)amino]-2-hydroxy-1-(phénylméthyl)propyl]-2-méthyl-3-(méthylsulfonyl)-, [1S-[1R*(R*),2S*]]-
